# EUROPEAN PATENT SPECIFICATION

(11) **EP 3 607 986 B1**
(45) Date of publication and mention of the grant of the patent: **12.02.2025**
(21) Application number: 19197088.8
(22) Date of filing: 03.12.2013
(51) Int. Cl.: A61M 15/00

(54) **DEVICES AND METHODS FOR PUNCTURING A CAPSULE TO RELEASE A POWDERED MEDICAMENT THEREFROM**
VORRICHTUNGEN UND VERFAHREN ZUR PUNKTIERUNG EINER KAPSEL ZUR FREISETZUNG EINES PULVERFÖRMIGEN MEDIKAMENTS DARAUS
DISPOSITIFS ET PROCÉDÉS PERMETTANT DE PERFORER UNE CAPSULE POUR LIBÉRER UN MÉDICAMENT EN POUDRE À PARTIR DE CELLE-CI

(30) Priority: 04.12.2012 US 201261733117 P; 19.12.2012 US 201213719598
(43) Date of publication of application: 12.02.2020
(62) Divisional of application: 16167547.5
(73) Proprietor: Merz Pharmaceuticals, LLC, Raleigh, NC 27615 (US)
(72) Inventor: ELLWANGER, Colleen, Lincoln, RI 02865 (US); NOBLE, Brian, Havard, MA 01451 (US); COKER, Tim, Nashua, NH 03062 (US); PLUNKETT, Sean, Wesborough, MA 01581 (US)
(74) Representative: Carpmaels & Ransford LLP

(56) References cited:
- EP-A1- 0 385 156
- EP-A1- 0 666 085
- EP-A1- 1 654 966
- WO-A1-03/080163
- WO-A2-02/083220
- WO-A2-2008/156586
- WO-A2-2011/031294
- US-A- 3 518 992
- US-A- 5 472 719
- US-A1- 2003 131 847
- US-A1- 2004 206 350
- US-A1- 2005 056 280
- US-A1- 2010 300 440
- US-A1- 2011 220 106
- US-A1- 2012 111 325

## Description

### Cross-Reference to Related Applications

The present application claims priority to and the benefit of co-pending U.S. Patent Application No. 13/719,598, which was filed on December 19, 2012, and co-pending U.S. Provisional Patent Application No. 61/733,117, which was filed on December 4, 2012.

### Technical Field

In various embodiments, the present invention relates to devices and methods for puncturing a capsule to release a powdered medicament therefrom.

### Background

In the medical field, it is often desirable to administer various forms of medication to patients. Well known methods of introducing medication into the human body include, for example, the oral ingestion of capsules and tablets, and intravenous injection through hypodermic needles. In accordance with another exemplary method, medications are inhaled into a patient's respiratory tract and lungs through the nose or mouth. Certain ones of these medications, such as those for the treatment of asthma and/or other respiratory anomalies (e.g., bronchodilators, corticosteroids, etc.), may be aimed at the respiratory tract directly. Others may be inhaled for purposes of systemic treatment, i.e., for treatment of any area of the body through absorption from the respiratory tract through the lung tissue, into the deep lungs, and into the bloodstream. Each of these medications comes in a variety of forms, including fluids, which are commonly administered as an aerosol vapor or mist, as well as solids. Inhalable solids typically take the form of fine, dry powders. Specialized devices, such as inhalers, may be provided to assist the patient in directing these fine powder medications into the respiratory tract.
US 2005/056280 A1 describes a method of aerosolizing a pharmaceutical formulation, which comprises providing an aerosolization device comprising an aerosolization chamber and providing a receptacle containing a pharmaceutical formulation. The receptacle comprises a wall having a weakened portion that opens when a force is applied. By applying a force to the receptacle to create an opening at the weakened portion, the pharmaceutical formulation in the receptacle is exposed so that it may be aerosolized for delivery to a patient's respiratory tract.
WO 03/080163 A1 describes a puncturing device for puncturing of a wall, in particular the wall of a capsule containing medication for inhalation. The puncturing device or assembly comprises one or more substantially longitudinal prongs, each having a puncturing surface at its distal end as well as a primary cutting edge disposed on the periphery of the prong and terminating at the puncturing surface. A substantially planar face is disposed on the periphery of each prong opposite of the primary cutting edge.

Various types of inhalers are known for the administration of dry powder medicaments. Typically, the dry powder medicament is initially contained in a capsule. In order for the powder to be emitted from the capsule, the inhaler must first create a passage through the capsule film. This is generally done through the use of sharpened pins or staples that pierce the capsule. In particular, the capsule film is typically thin and made of a material that has relatively low strength properties, thereby facilitating the piercing of the capsule.

Generally, 20 mg to 30 mg of a traditional inhalation powder made through dry blending of an active drug substance with lactose carrier particles are included in a capsule. The volume of this powder is typically low, however, due to the density of the powder generally being on the order of 1 g/cm³. Because the volume is low, the required capsule size is also small. For example, a lactose blend product can be easily accommodated in a size 3 (i.e., 0.30 cm³) or lower (i.e., smaller) capsule. In practice, however, the final decision on capsule size is more often than not related to patient convenience than to the volumetric requirements, as capsules that are too small can be difficult for patients to handle.

In cases where a low volume of powder is to be delivered, the required volumetric flow rate of the powder (i.e., the required volume of powder delivered per unit time) through one or more openings created in the capsule is also very modest. For example, with a powder density of approximately 1 g/cm³, a 25 mg fill of a lactose blend with a total active drug load of 0.20 mg has a volume of approximately 0.025 cm³. In this example, for a 5 second inhalation, the required volumetric flow rate is just 0.005 cm³/s.

However, high performance inhalation powders have recently been introduced as an alternative to traditional lactose blends. These new powders are characterized by highly efficient delivery of drug to the lungs, which is generally achieved by producing powders with low densities (i.e., typically below 0.10 g/cm³). These lower density, high performance powders create new demands on the delivery devices used by patients.

One consideration is that larger capsules are required. For example, 25 mg of powder with a density of 0.04 g/cm³ has a volume of 0.625 cm³. This volume of powder requires at least a size 0 (i.e., 0.68 cm³) capsule, and possibly even a size 00 (i.e., 0.95 cm³) capsule to allow for a reasonable commercial filling process.

Another consideration is that a full dose emission should be achievable in a single breath of a typical adult patient. As described above, the volumetric flow rate required for traditional dry powder blends is very modest. In comparison, a size 00 (i.e., 0.95 cm³) capsule with a 25 mg fill of a 0.04 g/cm³ powder (i.e., 0.625 cm³ of powder) requires a volumetric flow rate of 0.125 cm³/s in order to be fully emitted during a 5 second inhalation, which is 25 times greater than that required in the example provided above for lactose blends.

Small diameter pins or staples can readily pierce a capsule without causing undue material deformation, such as collapse of the capsule's walls or domes. For higher density lactose blends, use of small diameter pins or staples does not present an issue. In particular, the low volumetric flow rates required for these products allows for the total hole area to be small. The hole made by, for example, a 1 mm diameter round pin will have an area of about 0.008 cm². In the first (i.e., high density powder) example above, 25 mg of the 1 g/cm³ lactose blend powder emitted from a hole of this size in 5 seconds will have a volumetric flux of about 0.625 cm³/[cm²s]. This level of flux is readily obtainable in capsule-based inhalers. In the second (i.e., low density powder) example above, though, 25 mg of the 0.04 g/cm³ powder emitted from a 1 mm diameter hole in 5 seconds would require a volumetric flux of about 15.625 cm³/[cm²s]. In practice, a volumetric flux of this magnitude is not achievable. This can be remedied by increasing the hole area, but piercing a large hole through the capsule requires high force loading which will, without more, collapse the capsule before the puncture is created. Improving the sharpness of the piercing mechanism can also provide some relief, but this is limited by the nature of the metals and forming processes used.

Accordingly, a need exists for improved devices and methods for puncturing a capsule to release a powdered medicament therefrom. In particular, an improved approach is required in order to produce enough hole area in a capsule to allow for a full dose emission of a low density powder without the capsule being collapsed.

### Summary of the Invention

Various embodiments of the inhalation device described herein allow for high doses of low-density inhalation powders to be delivered. In one embodiment, the inhalation device accomplishes this by strategically piercing the highest strength region of the capsule (i.e., the domes) and by positioning the piercing elements towards the perimeter of the domed regions. In other words, the piercing elements (e.g., the individual prongs or tines) are placed far apart and at the point where most of their force is transmitted to the cylindrical wall of the capsule, thus placing as little force as possible on the dome. Such a design allows for relatively large pins or staple tines to produce large openings in the capsule's dome without collapsing the capsule. In particular, the inhalation device can incorporate pins or staples with large cross-sectional areas, which results in a substantial increase in the total hole area available for dose emission from the capsule.

According to the invention the location for the center of each puncture hole is in an annular region on the dome's surface that is positioned at no less than 40% (e.g., between about 40% and about 80%, or between about 40% and about 60%) of the dome's radius away from a central axis of the dome. In addition, in one such embodiment, the preferred total surface area of all puncture holes is between about 0.5% and about 2.2% of the total surface area of the capsule. It has been determined that these particular combinations of puncture hole location and puncture hole surface area advantageously avoid the capsule collapsing upon itself when punctured. Moreover, it has been determined that such a puncture hole surface area allows for a full dose of a low-density (i.e., below 0.10 g/cm³) powder to be emitted from a capsule at a sufficient volumetric flow rate and an achievable magnitude of volumetric flux so as to be consumed in a single breath by a typical adult patient.

In general, in one aspect, embodiments of the invention feature a device for puncturing a capsule to release a powdered medicament therefrom. The device includes a chamber for receiving the capsule. The capsule includes opposing domes and a cylindrical wall portion defined by a capsule wall radius r. The device further includes a mechanism for puncturing at least one hole in at least one dome. A center of each hole is located within an annular puncture region situated at no less than 0.4r, and a total surface area of all puncture holes is between about 0.5% and about 2.2% of a total surface area of the capsule. The annular puncture region is situated between 0.4r and 0.8r, or between 0.4r and 0.6r.

In general, in another aspect, embodiments of the invention feature a method for puncturing a capsule to release a powdered medicament therefrom. The method includes receiving, within a chamber, a capsule that itself includes opposing domes and a cylindrical wall portion defined by a capsule wall radius r. The method also includes puncturing at least one hole in at least one dome. A center of each hole is located within an annular puncture region situated at no less than 0.4r, and a total surface area of all puncture holes is between about 0.5% and about 2.2% of a total surface area of the capsule. The annular puncture region is situated between 0.4r and 0.8r, or between 0.4r and 0.6r.

In various embodiments, the puncturing mechanism (which may include a plurality of prongs and which may be moveable between a non-puncturing position and a puncturing position) is configured to puncture only a single dome. In such instances, the total surface area of all puncture holes is between about 3% and about 15% of a total surface area of the single dome. In one embodiment, the capsule has a volume of at least 0.50 cm³. The capsule may house a powdered medicament, which may have a density below 0.10 g/cm³ and/or contain levodopa as an active drug. Puncturing the capsule's dome causes the powdered medicament to be released from the capsule.

In certain embodiments, an outer surface of the capsule is between about 0.08 mm and about 0.12 mm thick. The capsule (i.e., the opposing domes and the cylindrical wall portion thereof) may be made from a material such as, for example, hydroxy propyl methyl cellulose or gelatin.

**In** one embodiment, the device further includes an inhalation portion that is coupled to the chamber. The inhalation portion may define, for example, at least one aperture for emitting the powdered medicament therethrough. For its part, the chamber may include a wall defining a plurality of vents for introducing air into the chamber to disperse the powdered medicament released from the capsule.

**In** general, in yet another aspect, embodiments, which are not explicitly recited by the wording of the claims, feature a punctured capsule. The punctured capsule includes opposing domes (at least one of which is punctured with at least one hole) and a cylindrical wall portion defined by a radius r. A center of each hole is located within an annular region situated at no less than 0.4r, and a total surface area of all puncture holes is between about 0.5% and about 2.2% of a total surface area of the capsule. The annular region is situated between 0.4r and 0.8r, or between 0.4r and 0.6r.

In various embodiments, only a single dome of the capsule is punctured. In such instances, the total surface area of all puncture holes is between about 3% and about 15% of a total surface area of the single dome. In one embodiment, the punctured capsule has a volume of at least 0.50 cm³. The punctured capsule may include therein a powdered medicament, which may have a density below 0.10 g/cm³ and/or contain levodopa as an active drug. In addition, an outer surface of the punctured capsule may be between about 0.08 mm and about 0.12 mm thick. The opposing domes and the cylindrical wall portion of the punctured capsule may each be made from a material such as, for example, hydroxy propyl methyl cellulose or gelatin.

These and other objects, along with advantages and features of the embodiments of the present invention herein disclosed, will become more apparent through reference to the following description, the accompanying drawings, and the claims. Furthermore, it is to be understood that the features of the various embodiments described herein are not mutually exclusive and can exist in various combinations and permutations.

### Brief Description of the Drawings

In the drawings, like reference characters generally refer to the same parts throughout the different views. Also, the drawings are not necessarily to scale, emphasis instead generally being placed upon illustrating the principles of the invention. In the following description, various embodiments of the present invention are described with reference to the following drawings, in which:
FIG. 1 schematically illustrates a front view of an inhalation device in accordance with one embodiment of the invention;
FIG. 2 is a cross-sectional view of the exemplary device depicted in FIG. 1 along the line 2-2;
FIG. 3 is a table of standard capsule sizes;
FIG. 4 schematically illustrates a side view of a capsule in accordance with one embodiment of the invention;
FIG. 5 schematically illustrates a top view of a capsule's dome in accordance with one embodiment of the invention;
FIG. 6 is a table showing the percentage of powder emitted for various surface areas of puncture holes in a capsule;
FIG. 7 is a graph illustrating the percentage of powder emitted for various surface areas of puncture holes in a capsule;
FIG. 8 is a table showing the amount of deflection in a capsule's dome for various locations of a puncture hole's center in the capsule's dome; and
FIG. 9 is a graph illustrating the amount of deflection in a capsule's dome for various locations of a puncture hole's center in the capsule's dome.

### Description

In various embodiments, the present invention features devices and methods for puncturing a capsule to release a powdered medicament therefrom. In particular, the capsule is punctured in a specific region with sufficiently-sized puncture holes so as to allow a full dose of a low-density (i.e., below 0.10 g/cm³) powder to be emitted from the capsule and be consumed by a typical adult patient in a single breath (i.e., emitted at a sufficient volumetric flow rate and an achievable magnitude of volumetric flux), while, at the same time, not causing the capsule to collapse upon itself.

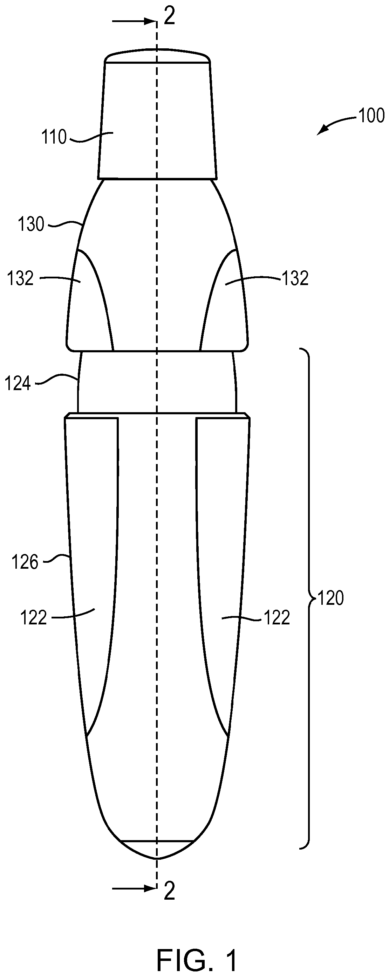

FIG. 1 depicts a front view of an inhalation device 100 in accordance with one embodiment of the invention. A rear view of the device 100 is substantially identical to the front view. As shown, the device 100 includes a first or lower casing portion 120 and a second or upper casing portion 130 removably coupled to the first casing portion 120. The upper casing portion 130 and lower casing portion 120 each include a flattened region 132 and 122, respectively, to facilitate gripping of the casing by a patient. In one embodiment, the lower casing portion 120 includes an outer casing 126 and an inner casing 124 movably received within the outer casing 126. A removable cap 110 is provided at the user or inhalation end of the device 100.

Preferred materials for the device 100 include Food and Drug Administration ("FDA") approved, and United States Pharmacopeia ("USP") tested, plastics. In one embodiment, the device 100 is manufactured using an injection molding process, the details of which would be readily apparent to one of ordinary skill in the art.

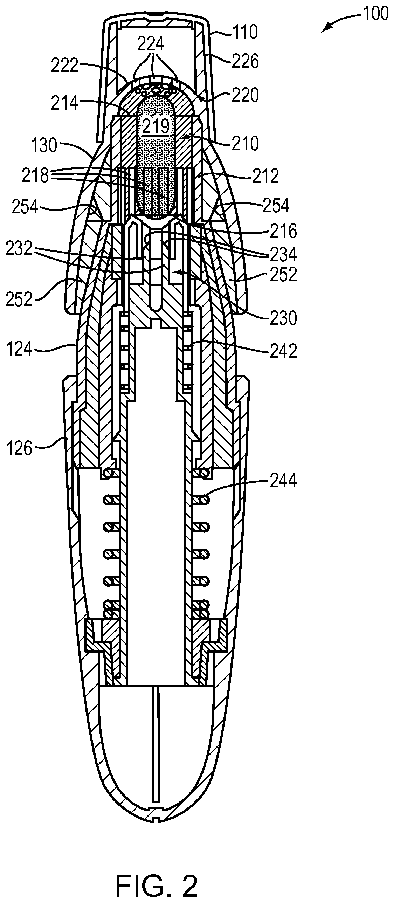

FIG. 2 is a cross-sectional view of the device 100 depicted in FIG. 1 along the line 2-2. As shown in FIG. 2, the device 100 includes an inhalation or emitter portion 220. The inhalation portion 220 includes a hemispheric region 222 that defines a plurality of apertures 224. It should be understood, however, that the present invention is not limited to a particular number of apertures 224, and can be configured such that at least one aperture 224 is provided. An inhalation piece 226 is provided to allow for inhalation of the medicament by a user. The inhalation piece 226 can be configured as a mouth piece for inhalation through a user's mouth. Alternatively, the inhalation piece 226 can be configured as a nose piece for inhalation through a user's nose.

The device 100 also includes a cylindrical chamber 210 that is defined by a straight wall 212 of circular cross-section. The chamber 210 has a proximal end 214 that is coupled to the inhalation portion 220, and an opposite, distal end 216. In particular, the proximal end 214 of the chamber 210 is in fluid communication with the inhalation portion 220. As shown in FIG. 2, the chamber 210 may receive therein a capsule 219. A plurality of vents 218 are defined by the wall 212, and are configured for introducing air into the chamber 210 to disperse powdered medicament released from the capsule 219. It should be understood that the present invention is not limited to a particular number of vents 218, and can be configured such that at least one vent 218 is provided. Powder released from the capsule 219 is dispersed in the chamber 210 and inhaled through the apertures 224 and inhalation piece 226 by the user.

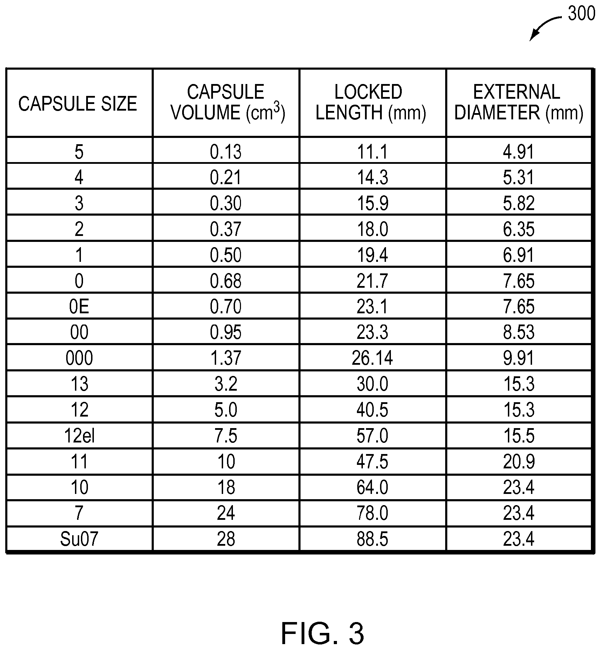

FIG. 3 depicts a table 300 of standard capsule sizes. In one embodiment of the invention, the capsule 219 employed in connection with the inhalation device 100 has a volume of at least 0.50 cm³. In other words, with reference to the table 300 of FIG. 3, a size 1 capsule is the minimum capsule size employed. Alternatively, the capsule 219 may be at least of size 0 (i.e., 0.68 cm³), size 0E (i.e., 0.70 cm³), size 00 (i.e., 0.95 cm³), or size 000 (i.e., 1.37 cm³). Suitable capsules 219 can be obtained, for example, from Shionogi, Inc. of Florham Park, New Jersey.

In one embodiment, the capsule 219 stores or encloses particles, also referred to herein as powders. The capsule 219 may be filled with powder in any manner known to one skilled in the art. For example, vacuum filling or tamping technologies may be used. In one embodiment, the capsule 219 is filled with a powdered medicament having a density below 0.10 g/cm³. The powdered medicament housed by the capsule 219 may also include any of a variety of active drugs, including, for example, levodopa. In one embodiment, the powder housed within the capsule 219 has a mass of at least 20 mg. In another embodiment, the mass of the powder is at least 25 mg, and up to approximately 30 mg.

With reference again to FIG. 2, the inhalation device 100 also includes a puncturing mechanism 230 that is used to puncture at least one hole in at least one dome of the capsule 219 to release the powdered medicament contained therein into the chamber 210. In the embodiment shown in FIG. 2, the puncturing mechanism 230 is configured as a substantially U-shaped staple having two prongs 232. In one such embodiment, each of prongs 232 is configured with a square cross-section 234, thereby providing a sharp point and two cutting edges. Alternatively, one, or a plurality of, straight needle-like implements may be used as the puncturing mechanism 230. Further exemplary puncturing mechanisms suitable for use in connection with the inhalation device 100 are described in detail in, for example, United States Patent No. 6,732,732 and United States Patent Application Publication No. 2009/0025721, the disclosures of which are hereby incorporated herein by reference in their entireties. The puncturing mechanism 230 can be configured to puncture one or, alternatively, multiple hole(s) (through a single or, alternatively, multiple piercing point(s)) in the capsule 219. As described below, however, the total surface area of all puncture holes is of greater importance than the actual number of puncture holes.

The puncturing mechanism 230 is preferably configured to be movable between a non-puncturing position (as depicted in FIG. 2) and a puncturing position. In the puncturing position, the prongs 232 pierce or puncture the capsule 219 to make holes therein. In one embodiment, a biasing mechanism is provided that biases the puncturing mechanism 230 in the non-puncturing position. In the embodiment shown in FIG. 2, the biasing mechanism is configured as a first spring 242 that biases the substantially U-shaped staple 230 in the non-puncturing position.

As noted above with reference to FIG. 1, the lower casing portion 120 of the device 100 includes the inner casing 124 and the outer casing 126. As shown in FIG. 2, a second spring 244 is disposed in the lower casing portion 120. The second spring 244 biases the inner casing 124 in an outward position. Upon compression of the second spring 244, the inner casing 124 moves from the outward position to an inward position, thereby drawing the lower casing portion 120 toward the upper casing portion 130. Compression of the second spring 244 also causes compression of the first spring 242, thereby causing the puncturing mechanism 230 to move upward to the puncturing position and to pierce or puncture the capsule 219 to make holes therein. Upon release of compression, the first and second springs 242, 244 return to their biased state, thereby returning the puncturing mechanism 230 to its non-puncturing position, and the inner casing 124 to its outward position. In particular, upon the release of compression, the capsule 219 is stripped from the prongs 232 of the puncturing mechanism 230 as the first spring 242 returns to its biased state. The second spring 244 may act as a backup to strip the capsule 219 from the prongs 232 of the puncturing mechanism 230 in the event that the first spring 242 fails to do so.

Although the puncturing mechanism 230 of the inhalation device 100 depicted in FIG. 2 is configured to puncture only a single dome of the capsule 219, other designs are also within the scope of the invention. For example, as will be understood by one of ordinary skill in the art, the puncturing mechanism 230 may also be designed to (or a second puncturing mechanism may be employed to) puncture both domes of the capsule 219.

As also depicted in FIG. 2, a pair of flanges 252 is disposed on the lower casing portion 120. A pair of grooves 254 is disposed on the upper casing portion 130, so that the flanges 252 can be received within the grooves 254 to thereby couple the lower and upper casing portions 120, 130. In one embodiment, the lower and upper casing portions 120, 130 are coupled with a friction-fit engagement. A friction-fit engagement may be achieved using the groove 254 and flange 252 arrangement depicted in FIG. 2. Other alternative configurations for a friction-fit engagement will be readily apparent to one skilled in the art.

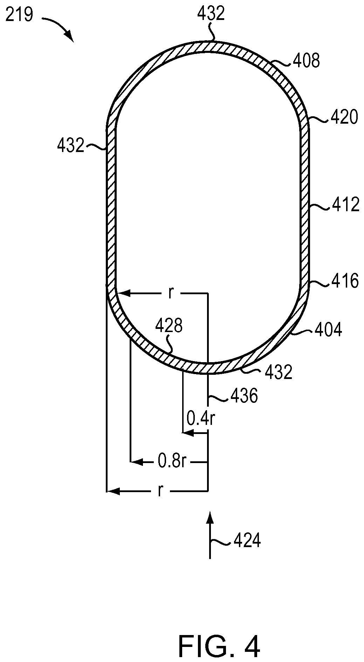

FIG. 4 depicts a side view of a capsule 219 that may be punctured using the exemplary inhalation device 100 described above. As illustrated, the capsule 219 includes a first dome 404, a second, opposing dome 408, and a cylindrical wall portion 412 that is defined by a radius r. The cylindrical wall portion 412 extends between first and second ends 416 and 420, where it meets the first and second domes 404 and 408, respectively.

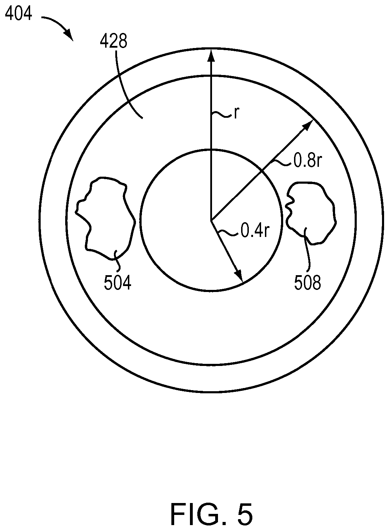

FIG. 5 depicts a top view of the first dome 404 (i.e., a view of the dome 404 when it is observed in the direction of arrow 424). As illustrated, the first dome 404 features two puncture holes 504, 508 within an annular region 428. As described further below, the annular puncture region 428 represents the preferred region on an outer surface 432 of the first dome 404 in which to puncture the holes 504, 508. In particular, in one embodiment, the puncturing mechanism 230 of the inhalation device 100 is configured to puncture a center of each hole 504, 508 within the annular puncture region 428.

In one embodiment, the outer surface 432 of the capsule 219 is between about 0.08 mm and about 0.12 mm thick. For example, the outer surface 432 of each of the first dome 404, the second dome 408, and the cylindrical wall portion 412 may be approximately 0.10 mm thick. Within that outer surface 432 the capsule 219 may be hollow and, as described above, may be at least partially filled with a powdered medicament. Materials such as, for example, hydroxy propyl methyl cellulose or gelatin may form the relatively thin outer surface 432 of the capsule 219 (i.e., the opposing domes 404 and 408 and the cylindrical wall portion 412).

As illustrated in FIGS. 4 and 5, the annular puncture region 428 is situated on the outer surface 432 of the first dome 404 between about 0.4r and about 0.8r. In other words, the preferred location for the center of each puncture hole 504, 508 is in an annular region of the dome 404 that is positioned between about 40% and about 80% of the dome's radius away from a central axis 436 of the dome 404. As an example, for a size 00 (i.e., 0.95 cm³) capsule 219, the annular puncture region 428 is situated between about 0.16 cm and about 0.32 cm away from the central axis 436 of the dome 404. It has been found that, in puncturing the dome 404 in this region 428, most of the force is transmitted to the cylindrical wall 412 of the capsule 219, thus placing as little force as possible on the dome 404. Such an approach allows for the use of relatively large prongs 232 in the puncturing mechanism 230 so as to produce large holes 504, 508 in the dome 404 without collapsing the capsule 219.

In particular, where the puncturing mechanism 230 is configured to puncture only a single dome 404 of the capsule 219 (as is the case, for example, in the exemplary inhalation device 100 depicted in FIG. 2), the total combined surface area of all puncture holes 504, 508 may be up to about 15% of a total surface area of the dome 404. As an example, each puncture hole 504, 508 may represent about 7.5% of the total surface area of the dome 404, and, thus, in combination the puncture holes 504, 508 may represent about 15% of the total surface area of the dome 404. This is a substantial total hole area that is available for dose emission from the capsule 219.

In fact, in testing, it has been found that a full dose of a low-density (i.e., below 0.10 g/cm³) powder may be emitted from the capsule 219 and consumed by a typical adult patient in a single breath (i.e., emitted at a sufficient volumetric flow rate and an achievable magnitude of volumetric flux) where the combined total surface area of all puncture holes is between about 3% and about 15% of a total surface area of a single dome 404 or, equivalently, where the combined total surface area of all puncture holes is between about 0.5% and about 2.2% of a total surface area of the entire capsule 219. As an example, for a size 00 (i.e., 0.95 cm³) capsule 219, the preferred total surface area for all puncture holes 504, 508 is between about 0.03 cm² and 0.14 cm².

### Experimental Results and Simulation

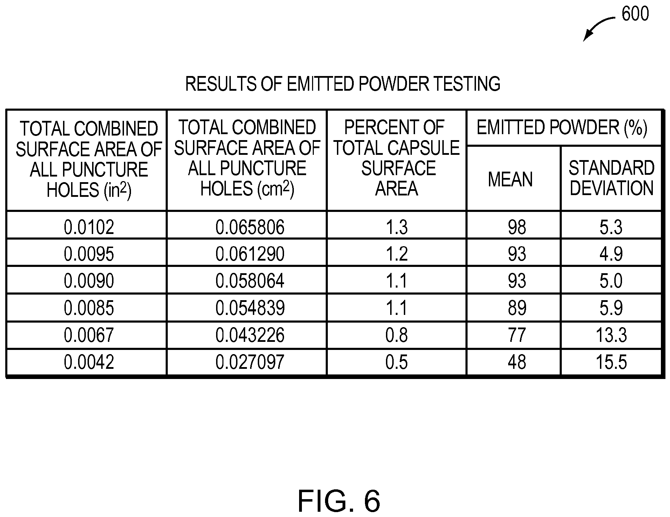

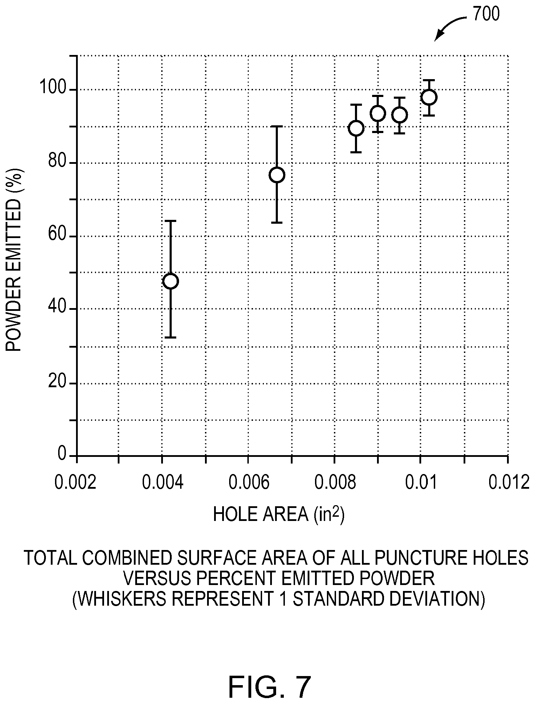

The effect of the total combined surface area of all puncture holes on the efficiency of dose delivery was examined using a representative low density, high performance dry powder formulation. In particular, size 00 (i.e., 0.95 cm³) capsules were filled with equal quantities of powder and punctured in a manner so as to create holes with a total combined surface area ranging from 0.027 cm² to 0.066 cm² (i.e., 0.0042 in² to 0.0102 in²). Approximately 30 capsules were tested for each target hole area value. The percentage of the filled powder mass emitted during a simulated breath was then measured for each hole area configuration. Specifically, this dose emission study was conducted at a simulated inhalation flow rate and volume performance associated with typical pediatric patients. The study therefore represents the worst case in adult populations (i.e., the study is representative of the lower 5% to 10% of adults). The results of the study are shown in the table 600 of FIG. 6 and in the corresponding graph 700 of FIG. 7.

From the results shown in FIGS. 6 and 7, it was concluded that the average fraction of powder emitted in a single breath increases asymptotically towards 100% with increasing puncture hole area. In addition, the variability of dose emission follows an inverse relationship with the total combined surface area of all puncture holes, as the standard deviation (a measure of dose delivery variability) decreases with increasing puncture hole area.

In particular, as can be seen in the table 600 depicted in FIG. 6, when a combined total surface area of all the puncture holes is about 0.5% of the total surface area of the entire capsule, 48% of the capsule's powder is emitted, on average, in a single breath of a pediatric patient. This represents the lower bound on an acceptable percentage of powder to be emitted in a single breath of a pediatric patient. In a typical adult, a much greater percentage of powder (e.g., essentially a full dose) would be emitted when the combined total surface area of all the puncture holes is about 0.5% of the total surface area of the entire capsule. This minimum value of surface area for the puncture holes therefore also represents the lower bound on an acceptable percentage of powder to be emitted in a single breath of an adult patient.

While the percentage of powder emitted in a single patient breath increases with increasing puncture hole area, it does so generally asymptotically. It has been found that it is undesirable for the combined total surface area of all the puncture holes to be greater than about 2.2% of the total surface area of the entire capsule, because the puncturing force that results from producing puncture holes greater than that size can approach or exceed the loading limits for typical capsule materials, such as hydroxy propyl methyl cellulose and gelatin. Moreover, it is typically unnecessary for the combined total surface area of all the puncture holes to be greater than about 2.2% of the total surface area of the entire capsule because, as can be seen from the table 600 of FIG. 6 and the graph 700 of FIG. 7, the percentage of powder emitted from the capsule approaches 100% generally asymptotically and little to no appreciable benefit (in terms of the percentage of powder emitted from the capsule) exists for puncture hole areas beyond that size.

The use of puncture holes having a combined total surface area in narrower ranges between about 0.5% and about 2.2% of the total surface area of the entire capsule (e.g., with minimum values of about 0.5%, about 0.8%, about 1.1%, and/or about 1.3% of the total surface area of the entire capsule in any combination with maximum values of about 1.6%, about 1.8%, about 2.0%, and/or about 2.2% of the total surface area of the entire capsule) is also contemplated and within the scope of the present invention.

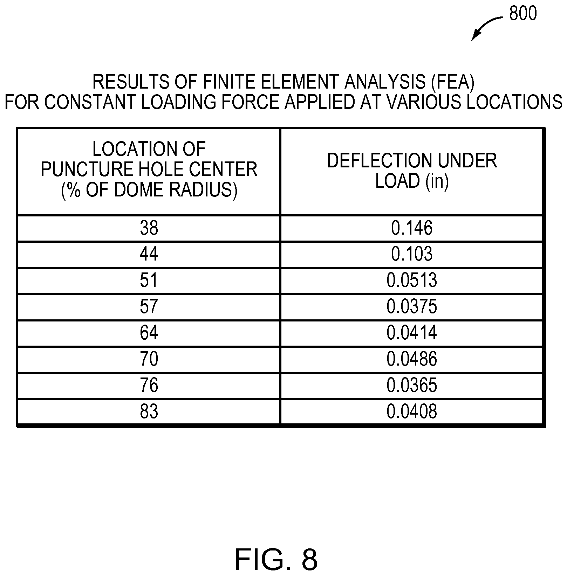

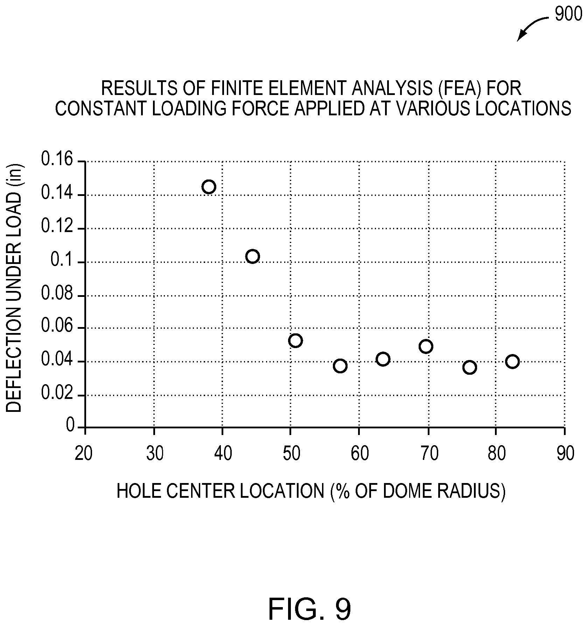

A limiting factor for positioning a puncture hole in a capsule's dome is the capsule material's strength and tendency to deflect under load. In order for the capsule material to be penetrated, the capsule material has to essentially maintain its position prior to the penetrating tip perforating the capsule's surface. If the capsule material deflects (e.g., bends inward) to too great a degree before perforation occurs, the capsule's dome will tend to collapse before the tip fully penetrates and creates a hole in the capsule material. Using Finite Element Analysis ("FEA") and the mechanical properties of the capsule material, the capsule material's response to a constant force loading at different positions along the radius of the capsule's dome was simulated. The results of that analysis are shown in the table 800 of FIG. 8 and in the corresponding graph 900 of FIG. 9.

The analysis predicts, as can be observed from FIGS. 8 and 9, that a change in degree of deflection in response to a constant loading force similar to that imparted to the capsule material during puncturing will occur between 40% to 50% of the dome radius. The change, as one moves from a puncture hole centered at 50% of the dome radius towards a puncture hole centered at 40% of the dome radius, is a transition from minor bending (which is recoverable or elastic deformation) to plastic or irreversible deformation. This transition occurs when the capsule material begins to yield under load. Once this transition point is reached, the efficiency of puncture hole generation is significantly reduced as the capsule's dome will continue to deflect under increasing load rather than being penetrated.

A separate laboratory study measuring the efficiency of puncture hole generation for various geometric positions of two penetrating tips was conducted to confirm these simulation results. The study showed that once the centers of the puncture holes reached values below 0.4r the rate of dome collapse increased dramatically. The nature of the dome collapse was such that a reliable dose emission was unlikely to occur with penetration positions at less than 0.4r.

Accordingly, as mentioned above, the preferred location for the center of each puncture hole is in an annular region of the capsule's dome that is situated at no less than 0.4r (and, in some embodiments, at no less than 0.5r). For example, the annular puncture region may be situated between about 0.4r and about 0.6r, or between about 0.4r and about 0.8r. In fact, in practice, the annular puncture region may be situated in any region on the capsule's dome having a minimum value of about 0.4r, about 0.5r, and/or about 0.6r in any combination with a maximum value of about 0.6r, about 0.7r, and/or about 0.8r. Attempting to puncture the capsule's dome in a region greater than 0.8r is undesirable for several reasons. For instance, beyond 0.8r the prong of the puncturing mechanism could slip off the capsule's dome and/or tear down the cylindrical wall portion of the capsule. Tearing down the cylindrical wall portion of the capsule could leave too great a hole in the capsule and/or cause portions of the capsule to be ripped apart and (potentially) be inhaled by the patient. Attempting to puncture the capsule's dome in a region greater than 0.8r could also create a side load on the capsule, causing it to detrimentally deflect within the inhaler's chamber.

### Exemplary Method of Use

In an exemplary method of use of the inhalation device 100, a user (e.g., a patient) places the capsule 219 containing a powdered medicament within the cylindrical chamber 210. When the user compresses the inhalation device 100, the puncturing mechanism 230 is moved toward the capsule 219, thereby puncturing the capsule 219 and causing the release of powdered medicament into the chamber 210. After release into the chamber 210, the powdered medicament is then inhaled by the user through the apertures 224 and the inhalation piece 226. As noted, the inhalation piece 226 can be configured as either a mouth piece or a nose piece. For subsequent uses, the user merely replaces the emptied capsule 219 with another capsule 219 that contains a new supply of the powdered medicament.

Having described certain embodiments of the invention, it will be apparent to those of ordinary skill in the art that other embodiments incorporating the concepts disclosed herein may be used without departing from the scope of the invention as defined by the appended claims. Accordingly, the described embodiments are to be considered in all respects as only illustrative and not restrictive.

## Claims

1. A device for puncturing a capsule to release a powdered medicament therefrom, the device comprising:
a chamber adapted to receive a capsule comprising opposing domes and a cylindrical wall portion defined by a capsule wall radius r; and
a puncturing mechanism adapted to puncture a received capsule by way of puncturing at least one hole in at least one dome of the received capsule, wherein a centre of each hole being punctured by the puncturing mechanism is located within an annular puncture region of the capsule situated between 0.4r and 0.8r,
wherein the puncturing mechanism is adapted to puncture a total surface area of all puncture holes between 0.5% and 2.2% of a total surface area of the received capsule.

2. A method for puncturing a capsule to release a powdered medicament therefrom, the method comprising:
receiving, within a chamber, a capsule comprising opposing domes and a cylindrical wall portion defined by a capsule wall radius r; and
puncturing with a puncturing mechanism at least one hole in at least one dome of the received capsule, a centre of each hole located within an annular puncture region situated between 0.4r and 0.8r,
wherein a total surface area of all puncture holes is between 0.5% and 2.2% of a total surface area of the capsule.

3. The device of claim 1, wherein the mechanism is configured to puncture only a single dome of the received capsule; or
the method of claim 2, wherein puncturing the at least one hole in the at least one dome comprises puncturing only a single dome.

4. The device or the method of claim 3, wherein the puncturing mechanism is adapted to puncture a total surface area of all puncture holes of between 3% and 15% of a total surface area of the single dome of the received capsule.

5. The device of claim 1, or the method of claim 2, wherein the chamber is adapted to receive a capsule having a volume of at least 0.50 cm³.

6. The device of claim 1, or the method of claim 2, wherein the puncturing mechanism is adapted to puncture a received capsule housing a powdered medicament comprising levodopa as an active drug.

7. The device of claim 1, or the method of claim 2, wherein the puncturing mechanism is adapted to puncture a received capsule housing a powdered medicament having a density below 0.10 g/cm³.

8. The device of claim 1, or the method of claim 2, wherein the puncturing mechanism is adapted to puncture a received capsule having an outer surface comprising a thickness between 0.08 mm and 0.12 mm.

9. The device of claim 1, or the method of claim 2, wherein the puncturing mechanism is adapted to puncture a received capsule having opposing domes and a cylindrical wall portion each comprising a material selected from the group consisting of hydroxy propyl methyl cellulose and gelatin.

10. The device of claim 1 further comprising an inhalation portion coupled to the chamber, the inhalation portion defining at least one aperture for emitting a powdered medicament therethrough.

11. The device of claim 1, wherein the chamber comprises a wall defining a plurality of vents for introducing air into the chamber to disperse a powdered medicament released from a capsule by way of the received capsule being punctured by the device.

12. The device of claim 1, wherein the puncturing mechanism comprises a plurality of prongs and is moveable between a non-puncturing position and a puncturing position.

13. The method of claim 2, wherein use of the puncturing mechanism to puncture the at least one hole in at least one dome of a received capsule causes a powdered medicament to be released from the received capsule.

## Patentansprüche

1. Vorrichtung zum Durchstechen einer Kapsel, um ein pulverförmiges Medikament daraus freizusetzen, wobei die Vorrichtung umfasst:
eine Kammer, ausgelegt zur Aufnahme einer Kapsel, die gegenüberliegende Kuppeln und einen zylindrischen Wandabschnitt, der durch einen Kapselwandradius r definiert ist, umfasst; und
einen Stechmechanismus, ausgelegt zum Durchstechen einer aufgenommenen Kapsel durch Stechen wenigstens eines Lochs in wenigstens eine Kuppel der aufgenommenen Kapsel, wobei ein Zentrum jedes durch den Stechmechanismus gestochenen Lochs innerhalb eines ringförmigen Durchstechbereichs der Kapsel, der zwischen 0,4 r und 0,8 r liegt, angeordnet ist,
wobei der Stechmechanismus dafür ausgelegt ist, eine Gesamtfläche aller gestochenen Löcher von zwischen 0,5 % und 2,2 % einer Gesamtoberfläche der aufgenommenen Kapsel zu stechen.

2. Verfahren zum Durchstechen einer Kapsel, um ein pulverförmiges Medikament daraus freizusetzen, wobei das Verfahren umfasst:
Aufnehmen einer Kapsel, die gegenüberliegende Kuppeln und einen zylindrischen Wandabschnitt, der durch einen Kapselwandradius r definiert ist, umfasst, in einer Kammer; und
Stechen wenigstens eines Lochs in wenigstens eine Kuppel der aufgenommenen Kapsel mit einem Stechmechanismus, wobei ein Zentrum jedes Lochs innerhalb eines ringförmigen Durchstechbereichs, der zwischen 0,4 r und 0,8 r liegt, angeordnet ist,
wobei eine Gesamtfläche aller gestochenen Löcher zwischen 0,5 % und 2,2 % einer Gesamtoberfläche der Kapsel beträgt.

3. Vorrichtung nach Anspruch 1, wobei der Mechanismus dafür gestaltet ist, nur eine einzige Kuppel der aufgenommenen Kapsel zu durchstechen; oder
Verfahren nach Anspruch 2, wobei das Stechen des wenigstens einen Lochs in die wenigstens einen Kuppel Durchstechen nur einer einzigen Kuppel umfasst.

4. Vorrichtung oder Verfahren nach Anspruch 3, wobei der Stechmechanismus dafür ausgelegt ist, eine Gesamtfläche aller gestochenen Löcher zwischen 3 % und 15 % einer Gesamtoberfläche der einzelnen Kuppel der aufgenommenen Kapsel zu stechen.

5. Vorrichtung nach Anspruch 1 oder Verfahren nach Anspruch 2, wobei die Kammer dafür ausgelegt ist, eine Kapsel mit einem Volumen von wenigstens 0,50 cm³ aufzunehmen.

6. Vorrichtung nach Anspruch 1 oder Verfahren nach Anspruch 2, wobei der Stechmechanismus dafür ausgelegt ist, eine aufgenommene Kapsel zu durchstechen, die ein pulverförmiges Medikament enthält, das Levodopa als Wirkstoff umfasst.

7. Vorrichtung nach Anspruch 1 oder Verfahren nach Anspruch 2, wobei der Stechmechanismus dafür ausgelegt ist, eine aufgenommene Kapsel, die ein pulverförmiges Medikament mit einer Dichte unter 0,10 g/cm³ enthält, zu durchstechen.

8. Vorrichtung nach Anspruch 1 oder Verfahren nach Anspruch 2, wobei der Stechmechanismus dafür ausgelegt ist, eine aufgenommene Kapsel mit einer Außenfläche, die eine Dicke zwischen 0,08 mm und 0,12 mm aufweist, zu durchstechen.

9. Vorrichtung nach Anspruch 1 oder Verfahren nach Anspruch 2, wobei der Stechmechanismus dafür ausgelegt ist, eine aufgenommene Kapsel mit gegenüberliegenden Kuppeln und einem zylindrischen Wandabschnitt, die jeweils ein Material ausgewählt aus der Gruppe bestehend aus Hydroxypropylmethylcellulose und Gelatine umfassen, zu durchstechen.

10. Vorrichtung nach Anspruch 1, ferner umfassend einen Inhalationsabschnitt, der an die Kammer gekoppelt ist, wobei der Inhalationsabschnitt wenigstens eine Öffnung zum Emittieren eines pulverförmigen Medikaments hindurch definiert.

11. Vorrichtung nach Anspruch 1, wobei die Kammer eine Wand umfasst, die eine Vielzahl von Lüftungsöffnungen zum Einführen von Luft in die Kammer definiert, um ein pulverförmiges Medikament zu zerstreuen, das aus einer Kapsel freigesetzt wird, indem die aufgenommene Kapsel durch die Vorrichtung durchstochen wird.

12. Vorrichtung nach Anspruch 1, wobei der Stechmechanismus eine Mehrzahl von Zinken umfasst und zwischen einer nichtstechenden Stellung und einer stechenden Stellung beweglich ist.

13. Verfahren nach Anspruch 2, wobei Verwendung des Stechmechanismus zum Stechen des wenigstens einen Lochs in wenigstens eine Kuppel einer aufgenommenen Kapsel bewirkt, dass ein pulverförmiges Medikament aus der aufgenommenen Kapsel freigesetzt wird.

## Revendications

1. Dispositif permettant de perforer une capsule pour en libérer un médicament en poudre, le dispositif comprenant :
une chambre adaptée pour recevoir une capsule comprenant des dômes opposés et une partie de paroi cylindrique définie par un rayon de paroi de capsule r ; et
un mécanisme de perforation adapté pour perforer une capsule reçue en perforant au moins un trou dans au moins un dôme de la capsule reçue, un centre de chaque trou perforé par le mécanisme de perforation étant situé dans une région de perforation annulaire de la capsule située entre 0,4r et 0,8r,
le mécanisme de perforation étant adapté pour perforer une surface totale de tous les trous de perforation comprise entre 0,5 % et 2,2 % de la surface totale de la capsule reçue.

2. Procédé de perforation d'une capsule pour en libérer un médicament en poudre, le procédé comprenant :
la réception, à l'intérieur d'une chambre, d'une capsule comprenant des dômes opposés et une partie de paroi cylindrique définie par un rayon de paroi de capsule r ; et
la perforation, à l'aide d'un mécanisme de perforation, d'au moins un trou dans au moins un dôme de la capsule reçue, un centre de chaque trou étant situé dans une région de perforation annulaire située entre 0,4r et 0,8r,
une surface totale de tous les trous de perforation étant comprise entre 0,5 % et 2,2 % d'une surface totale de la capsule.

3. Dispositif selon la revendication 1, le mécanisme étant configuré pour ne perforer qu'un seul dôme de la capsule reçue ; ou
procédé selon la revendication 2, la perforation de l'au moins un trou dans l'au moins un dôme comprenant la perforation d'un seul dôme.

4. Dispositif ou procédé selon la revendication 3, le mécanisme de perforation étant adapté pour perforer une surface totale de tous les trous de perforation comprise entre 3 % et 15 % d'une surface totale du dôme unique de la capsule reçue.

5. Dispositif selon la revendication 1, ou procédé selon la revendication 2, la chambre étant adaptée pour recevoir une capsule ayant un volume d'au moins 0,50 cm³.

6. Dispositif selon la revendication 1, ou procédé selon la revendication 2, le mécanisme de perforation étant adapté pour perforer une capsule reçue contenant un médicament en poudre comprenant de la lévodopa en tant que médicament actif.

7. Dispositif selon la revendication 1, ou procédé selon la revendication 2, le mécanisme de perforation étant adapté pour perforer une capsule reçue contenant un médicament en poudre ayant une densité inférieure à 0,10 g/cm³.

8. Dispositif selon la revendication 1, ou procédé selon la revendication 2, le mécanisme de perforation étant adapté pour perforer une capsule reçue ayant une surface extérieure comprenant une épaisseur comprise entre 0,08 mm et 0,12 mm.

9. Dispositif selon la revendication 1, ou procédé selon la revendication 2, le mécanisme de perforation étant adapté pour perforer une capsule reçue ayant des dômes opposés et une partie de paroi cylindrique comprenant chacun un matériau choisi dans le groupe constitué de l'hydroxy propyl méthyl cellulose et de la gélatine.

10. Dispositif selon la revendication 1, comprenant en outre une partie d'inhalation couplée à la chambre, la partie d'inhalation définissant au moins une ouverture pour émettre un médicament en poudre à travers celle-ci.

11. Dispositif selon la revendication 1, la chambre comprenant une paroi définissant une pluralité d'évents pour introduire de l'air dans la chambre afin de disperser un médicament en poudre libéré d'une capsule par la perforation de la capsule reçue par le dispositif.

12. Dispositif selon la revendication 1, le mécanisme de perforation comprenant une pluralité de dents et étant mobile entre une position de non-perforation et une position de perforation.

13. Procédé selon la revendication 2, l'utilisation du mécanisme de perforation pour perforer l'au moins un trou dans au moins un dôme d'une capsule reçue provoquant la libération d'un médicament en poudre de la capsule reçue.
